(19) Europäisches Patentamt / European Patent Office / Office européen des brevets

(11) **EP 4 775 227 A1**

(12) **EUROPEAN PATENT APPLICATION**
published in accordance with Art. 153(4) EPC

(43) Date of publication:
**15.07.2026 Bulletin 2026/29**

(21) Application number: **24869430.9**

(22) Date of filing: **29.10.2024**

(51) International Patent Classification (IPC):
**A61K 47/69** (2017.01)      **A61K 9/14** (2006.01)
**A61K 9/16** (2006.01)      **A61K 47/02** (2006.01)

(52) Cooperative Patent Classification (CPC):
**A61K 31/366; A61K 9/143; A61K 9/2009;
A61K 31/192; A61K 31/196; A61K 31/216;
A61K 31/337; A61K 31/403; A61K 31/405;
A61K 31/4422; A61K 31/502; A61K 31/57**

(86) International application number:
**PCT/CN2024/128187**

(87) International publication number:
**WO 2026/085904 (30.04.2026 Gazette 2026/18)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **24.10.2024  CN 202411494974**

(71) Applicant: **Pharmaease Tech Limited
Central, Sheung Wan (HK)**

(72) Inventor: **XU, Zhuo
Hong Kong (HK)**

(74) Representative: **Metida
Gyneju str. 16
01109 Vilnius (LT)**

(54) **METHOD FOR IMPROVING DRUG SOLUBILITY AND DRUG COMPOSITE MATERIAL HAVING IMPROVED SOLUBILITY**

(57)     A method for enhancing drug solubility and preparing a drug composite material with enhanced solubility, comprising: S1, raw material preparation: preparing a colloidal suspension of silicon dioxide in water, including silicon dioxide nanoparticles with diameters ranging from 2 to 100 nm; S2, preparation of high-density silanol group surface material: rapidly evaporating the solvent from the silicon dioxide colloidal suspension by controlling temperature and pressure, thereby retaining an ultra-high density of silanol groups on the surface, enhancing the surface's affinity for drug molecules and water, and forming a porous structure to increase the specific surface area; S3, drug loading: mixing the active drug component with the high silanol group surface material, and load the drug molecules onto the material using a drug loading process. By carefully controlling the size of the nanoparticles and the density of the silanol groups, the invention can effectively adsorb drug molecules under anhydrous conditions and rapidly desorb and release them in aqueous environments, thereby significantly enhancing the drug's solubility, potentially by two to three orders of magnitude.

FIG. 1A

**Description**

**Technical Field**

The present invention relates to pharmaceutical technology, particularly to a method for enhancing drug solubility and preparing a drug composite material with enhanced solubility.

**Background**

In the pharmaceutical industry, 60% - 70% of drug candidates fail clinical testing and commercialization due to low solubility. Additionally, the number of insoluble drug candidates is increasing due to their lipophilic properties, which exhibit good affinity for receptors and lipid membranes within the human body but also face challenges in dispersing in water. Thus, developing effective methods to enhance solubility is crucial for the research and development of new drugs and the improvement of existing drugs.

To address this critical issue, various methods have been developed, such as chemical modification, reduction of particle size, solid dispersions, lipid-based formulations, or mesoporous encapsulation. However, each of these methods has its own drawbacks. Chemical modification involves altering the molecular structure of drugs and is only suitable for certain drugs. Particle size reduction achieved through mechanical force or precipitation typically only shows minor solubility enhancements. Solid dispersions and lipid-based formulations face drug-specific limitations and stability issues. While mesoporous inorganic materials exhibit excellent stability, they often suffer from incomplete drug release issues. Therefore, the current state-of-the-art faces several challenges, such as limited applicability, low effectiveness, stability issues, and high costs. Consequently, there is a need for a new generation of technology that is universally applicable, efficient, stable, and cost-effective.

It should be noted that the information disclosed in the background section above is only for understanding the context of this application and may include information that does not constitute prior art known to ordinary persons skilled in the field.

**Disclosure of Invention**

The main objective of the present invention is to address the issues found in the prior art by providing a method for enhancing drug solubility and preparing a drug composite material with enhanced solubility.

To achieve the above objectives, the present invention adopts the following technical solutions:

In the first aspect of the present invention, a method for enhancing drug solubility includes the following steps:

S1. Raw Material Preparation: Prepare a colloidal suspension of silicon dioxide in water, including silicon dioxide nanoparticles with diameters between 2 to 100 nm;

S2. Preparation of High-Density Silanol Group Surface Material: Rapidly evaporate the solvent from the silicon dioxide colloidal suspension by controlling temperature and pressure, retaining an ultra-high density of silanol groups on the surface, enhancing the surface's affinity for drug molecules and water, and forming a porous structure to increase the specific surface area;

S3. Drug Loading: Mix the active drug components with the high-density silanol group surface material, and load the drug molecules onto the material using a drug loading process. Furthermore, the density of silanol groups on the surface of the silicon dioxide nanoparticles is above 10 OH/nm$^2$, to increase the adsorption capacity of the silicon dioxide nanoparticles for drug molecules.

Additionally, in Step S2, the evaporation is carried out using a vacuum evaporation process. In Step S3, the drug loading process includes grinding, pressing, and baking the mixture of the active drug component and the high-density silanol group surface material.

Moreover, in Step S3, the pressing and baking process involves placing the ground mixture into a mold, pressing it with a hydraulic press to form a tablet, and subsequently baking the tablet at an optimized temperature for a set duration.

Furthermore, the active drug components include one or more of the following: fenofibrate, rapamycin, diclofenac, isotretinoin, trimetazidine, simvastatin, olaparib, rosiglitazone, indomethacin, felodipine, flurbiprofen, paclitaxel, docetaxel, naproxen, ibuprofen, progesterone, ketoprofen, and ramipril.

In the second aspect of the invention, a drug composite material with enhanced solubility is prepared using the method described above.

The present invention offers several beneficial effects:

The invention proposes an innovative method to enhance drug solubility using a specially prepared porous silicon dioxide template, which has a high specific surface area and ultra-high silanol density, thereby significantly enhancing the adsorption capacity for drug molecules. By carefully controlling the size of nanoparticles and the density of silanol groups, the invention can effectively adsorb drug molecules under anhydrous conditions and rapidly desorb and release them in aqueous environments, potentially enhance drug solubility by two to three orders of magnitude. The method is simple, cost-effective, scalable, and the prepared drug composite material exhibits excellent stability and long-term storage capabilities, making it a promising new generation formulation technology in the pharmaceutical industry.

The invention's multifunctional capabilities offer broad prospects for future applications in drug delivery, catalysis, controlled drug release, environmental remediation, and sensor development. The method based on physical adsorption and desorption mechanisms avoids chemical modification of drug molecules, providing broader applicability and higher safety. Overall, the invention provides an efficient, stable, economical, and broadly applicable new technology for enhancing drug solubility.

Other beneficial effects of the embodiments of the present invention will be further discussed in the following sections.

**Brief Description of the Drawings**

- **Figure 1A** illustrates the physical properties, in vitro and in vivo solubility performance, and long-term stability of the solubilization formulation PE-07 based on nanoscale silicon dioxide particles, demonstrating its effectiveness in enhancing drug solubility.
- **Figure 1B** evaluates the solubilization effects of the PE-07 formulation on 18 poorly soluble active pharmaceutical ingredients (APIs) using UV-VIS spectroscopy, showing its broad applicability and significant solubility enhancement capabilities across different drug molecules.
- **Figure 2** presents adsorption isotherms and desorption kinetics, along with a schematic representation of APIs on the surface of PE-07 silicon dioxide.
- **Figure 3** depicts experiments assessing the impact of silanol density, examining the solubilization effects of materials with different silanol densities, and measuring the energy changes from API-silanol to water-silanol through simulations.

**Detailed Description**

The following detailed description of the embodiments of the invention emphasizes that the description provided is merely exemplary and not intended to limit the scope or application of the invention.

Typically, silicon dioxide surfaces possess silanol groups (Si-OH). These silanol groups demonstrate an affinity for water and organic functional groups including carboxyl, carbonyl, hydroxyl, and amide, which are commonly found in pharmaceutical compounds. However, the typical density of silanol groups is 4-6 $OH/nm^2$. This high affinity for active pharmaceutical ingredients (APIs) over water leads to APIs not being released into the water but instead adhering to the surface.

The present invention is based on a novel mechanism based on the adsorption and desorption from a silicon dioxide surface with an ultra-high density of silanol groups, proposing a new method to enhance solubility applicable to various drugs, potentially becoming the next-generation formulation technology. By increasing the density of silanol groups, the material's surface affinity for both APIs and water is enhanced. However, the interaction with water increases more significantly. Thus, a surface designed with ultra-high density silanol can strongly adsorb various drug molecules under anhydrous conditions. When introduced into a water environment, such a surface preferentially binds water molecules, causing the previously adsorbed drug molecules to desorb and release. This adsorption-desorption cycle can significantly enhance the dispersion of drug molecules in water, potentially increasing solubility by two to three orders of magnitude.

**Embodiment of the Invention Provides a Method for Enhancing Drug Solubility, Including the Following Steps:**

**Step S1. Raw Material Preparation:** Prepare silicon dioxide nanoparticles with diameters between 2 to 100 nm as raw materials. To ensure biocompatibility, FDA-recognized Generally Recognized as Safe (GRAS) silicon dioxide may be selected as the raw material.

In a preferred embodiment, the density of silanol groups on the surface of the silicon dioxide nanoparticles is above 10 $OH/nm^2$ to enhance the adsorption capacity for drug molecules.

**Step S2. Preparation of Porous Template:** Disperse the silicon dioxide nanoparticles in a solvent to form a colloidal suspension, and rapidly evaporate the solvent by controlling temperature and pressure, inducing aggregation of the silicon dioxide nanoparticles to form a porous matrix with a large specific surface area, serving as a porous template.

In a preferred embodiment, the evaporation in Step S2 utilizes a vacuum evaporation process.

**Step S3. Drug Loading:** Mix the active drug components with the porous template and load the drug molecules into the porous template using a drug loading process to form a drug composite material.

In a preferred embodiment, the drug loading process in Step S3 includes grinding, pressing, and baking the mixture of the active drug components and the porous template. Additionally, the pressing and baking process involves placing the ground mixture into a mold, pressing it with a hydraulic press to form a tablet, and subsequently baking the tablet at an optimized temperature for a set duration.

In some embodiments, the active drug components include (but are not limited to) fenofibrate, rapamycin, diclofenac, isotretinoin, trimetazidine, simvastatin, olaparib, rosiglitazone, indomethacin, felodipine, flurbiprofen, paclitaxel, docetaxel, naproxen, ibuprofen, progesterone, ketoprofen, and ramipril.

This embodiment also provides a drug composite material with enhanced solubility, prepared by the method of enhancing drug solubility described in any one of the aforementioned embodiments.

Further descriptions of specific embodiments and experimental validations of the present invention follow.

To achieve high silanol density while preventing interaction between adjacent silanol groups, silicon dioxide nanoparticles with diameters ranging from 2 to 100 nm are used as the raw material. These nanoparticles possess high-curvature convex surfaces that effectively increase the distance between neighboring silanol groups on the curved surfaces, thereby reducing their mutual interaction. Starting from an initial colloidal suspension, the particles are induced to aggregate by rapidly evaporating the solvent under precisely controlled temperature and pressure conditions. The resulting dry particles are firmly adhered together through Si-O-Si bonds, and the pores formed are capable of accommodating drug molecules.

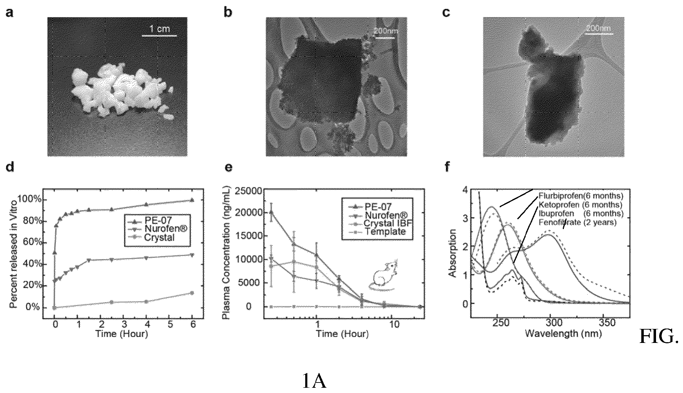

**Figure 1A** displays a solubilization formulation, PE-07, based on nanoscale silicon dioxide particles, which possesses competitive market advantages, in vivo validation, stability, and universality.

- (a) Actual photos of PE-07 excipients and
- (b) Microscopic TEM images where the nanoscale silicon dioxide particles are firmly connected, forming millimeter-scale solid blocks.
- (c) TEM images of the PE-07 formulation composite material, showing fenofibrate binding within the particle-filled porous template.
- In vitro (d) and in vivo (e) the solubility performance of PE-07 formulated ibuprofen (red triangles), Nurofen® (blue inverted triangles), and crystalline ibuprofen (yellow circles) are compared, with equal amounts of ibuprofen in each group.
- (f) Stability is assessed by comparing UV-VIS absorption of the original formulation (solid lines) and after storage (dashed lines) for four APIs: flurbiprofen (blue), ketoprofen (red), ibuprofen (black), and fenofibrate (purple). Flurbiprofen, ketoprofen, and ibuprofen were stored for six months, while fenofibrate was stored for two years.

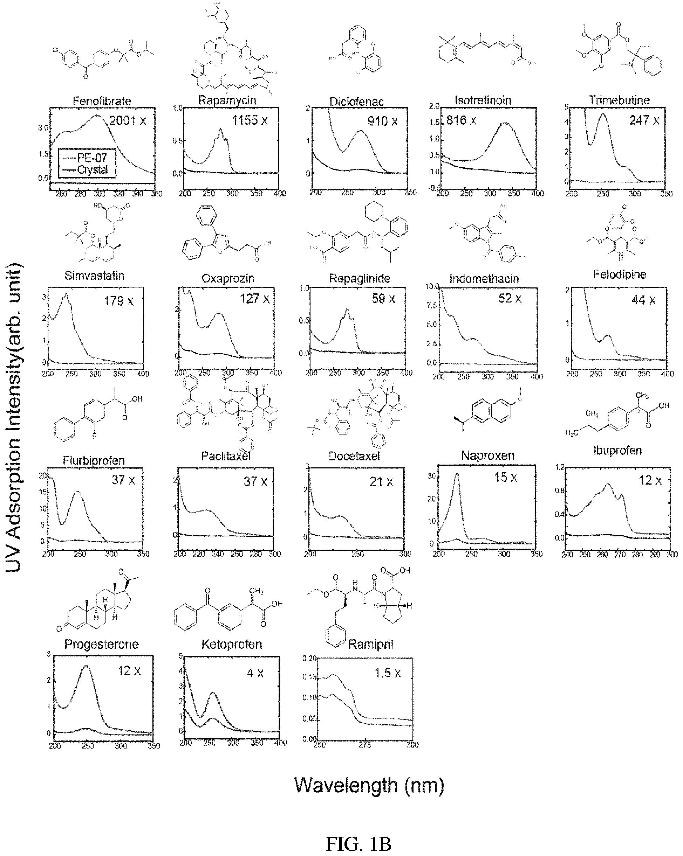

**Figure 1B** evaluates the solubilization effect of 18 poorly soluble APIs using UV-VIS spectroscopy by comparing the equilibrium concentrations of the crystalline form (black) and the PE-07 formulation (red), where the peak size of the spectra correlates linearly with the solution concentration. Measurements were taken after a dissolution period of 6 hours to ensure complete release of the APIs, using D.I. water as the solvent.

As depicted in **Figure 1A (a)-(b),** this porous matrix, referred to as a "template," possesses a large specific surface area and ultra-high silanol density, ranging from 10-30 OH/nm$^2$, which is 2-6 times that of traditional mesoporous silicon dioxide surfaces. The synergistic effect of a large surface area and ultra-high silanol density results in excellent adsorption capacity for a large number of drug molecules. Drug molecules can be loaded onto the porous template by various methods, such as injecting molten drug into the template or mixing drug powder with the template powder (method). By preparing the template and then loading the drug, the final drug composite material is produced, as shown in **Figure 1A (c).** The drug composite materials prepared by the present invention exhibit exceptionally high solubility in water, are cost-effective to produce, and demonstrate long-term stability-three key attributes of next-generation formulation technology. The specific performance and potential mechanisms of the present invention method will be detailed further below.

To assess the effectiveness of the invention, ibuprofen was incorporated as the active pharmaceutical ingredient (API) into a template constructed from 7 nm silicon dioxide particles (named PE-07). Comparative analyses were conducted via in vitro and in vivo experiments with the commercial ibuprofen product Nurofen® as a comparison.

For the in vitro dissolution tests, a standard dissolution testing apparatus was used, employing pure water as the dissolution medium to ensure a controlled test environment. The dissolution curves of the drug composite material from the present invention, the commercial product Nurofen®, and the crystalline form of the API are shown in Figure 1A(d). The results indicate that the composite material of the present invention achieves rapid dissolution, with solubility exceeding 90% within the first hour, whereas the crystalline API shows less than 20% solubility after 6 hours. Notably, the formulation of the invention demonstrated dissolution results that were twice as high as those of the established Nurofen® formulation.

To evaluate the in vivo effects, mice were used as test subjects in pharmacokinetic (PK) studies. Specifically, four groups of mice (six per group) were administered the composite material of the invention (PE-07), Nurofen®, crystalline API, and template, respectively, and their plasma concentrations were measured at various time points. As shown in Figure 1A(e), the PE-07 sample of the invention exhibited significantly enhanced pharmacokinetic properties: with the same amount of API, it achieved nearly twice the peak plasma concentration (Cmax) and more than 1.5 times the total exposure (i.e., area under the curve) compared to Nurofen® and crystalline API. These data highlight the superior performance of the composite material of the invention. Furthermore, the elevated plasma concentrations were rapidly reached within 15 minutes, indicating the fast action of the composite material of the invention. As expected, the template sample showed zero concentration, as it contained no API.

The long-term stability of the composite material of the invention was rigorously evaluated by comparing fresh samples with those stored for extended periods. As shown in Figure 1A(f), samples freshly prepared and those stored for 6 months and 2 years were assessed by dissolving them in pure water for 6 hours until reaching stable concentrations. Subsequently, the UV absorption spectra of each solution were measured, where peak height correlates directly with solute concentration. The results revealed significant consistency between freshly made and long-stored samples, confirming the enduring stability of the composite material of the invention.

Additionally, the broad applicability of the invention's method was tested by applying it to 18 various poorly soluble APIs, as shown in Figure 1B. Through UV spectroscopic comparison, data indicated that the PE-07 composite material (i.e., the template made from 7 nm nanoparticles) generally increased solubility by 10 to 2000 times compared to crystalline APIs.

In summary, all these data confirm that the method of the invention is not only effective and stable but also broadly applicable to various drugs. Moreover, the manufacturing process relies on commonly practiced techniques, namely temperature and pressure adjustments, which are simple, scalable, and cost-effective. Therefore, the technology of the present invention has the potential to become a next-generation formulation platform technology.

To elucidate the potential mechanism of the solubility enhancement technology of the present invention, a series of composite material samples were prepared, with each sample progressively loaded with increasing amounts of API into the template. Each sample was then dissolved in pure water, and the amount of API dissolved from each composite material was quantified. The experimental results showed that only a single layer of drug molecules coating the surface of the template dissolved in water. To ensure the broad applicability of the findings of the present invention, two different types of APIs, ibuprofen and fenofibrate, were used for this analysis, and the results are presented below.

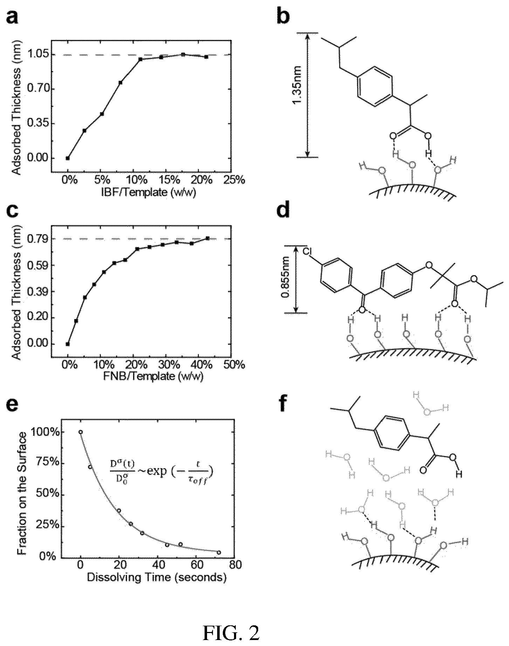

Figure 2 shows the adsorption isotherms and desorption kinetics, as well as a schematic diagram of APIs on the PE-07 silicon dioxide surface. The depth of dissolved API is plotted against the API/template mass ratio of the two model APIs, ibuprofen (a) and fenofibrate (c). The API/template ratio was adjusted by modifying the quantity of APIs while controlling the amount of the template. The depth of soluble API was calculated by dividing the volume of dissolved API by the surface area of the template, where the API volume was measured using UV-VIS spectroscopy after dissolution in water (see supplementary material for details). The illustrations demonstrate individual molecules, ibuprofen (b) and fenofibrate (d), adsorbed to the raised surface of PE-07 via hydrogen bonds (dashed lines), connecting APIs and Si-OH groups (highlighted in red). Annotations show the estimated molecular heights calculated using Bondi van der Waals radii. Part (e) shows the kinetics of the percentage of ibuprofen released in water. The data display a satisfactory logarithmic fit to the linearized form of the equation ln $\ln\left(\frac{D^\sigma(t)}{D_0^\sigma}\right) \sim -t$, where the power of time equals 1. In Part (f), the illustration explains the desorption of ibuprofen into the water medium, attributed to the competitive advantage of water (blue) over the surface.

As shown in Figures 2(a) and 2(c), although the amount of loaded API increases, the amount dissolved saturates to specific values, saturating at 11% mass fraction on the x-axis (Figure 2(a)) and 25% (Figure 2(c)), which correspond to volume fractions of 24% and 46%, respectively. These saturation points are far from the scenario where the template's pore space is fully occupied, (about 30% volume fraction, see measurements in Supplementary Information). Therefore, it is clear that the maximum amount of soluble API contained in the template is unrelated to the total pore volume of the template.

To understand the actual reason for this maximum solubility, the amount of dissolved API was converted into its corresponding thickness on the template surface. This was achieved by first determining the surface area of the porous template using the Brunauer-Emmett-Teller (BET) method (methodology and SI), then dividing the volume of dissolved API by this surface area. As shown in Figure 2(a), the thickness obtained for ibuprofen API is 1.05 nm, which is very close to the molecular height of 1.35 nm when ibuprofen molecules adsorb on the surface silanols, as shown in Figure 2(b).

A similar calculation was performed for fenofibrate. Figure 2(c) shows that it corresponds to a surface coating thickness of 0.79 nm, which again matches the molecular height of 0.855 nm when the API adsorbs to the surface, as shown in Figure 2(d). Notably, these two types of molecules adopt distinctly different orientations upon adsorption: ibuprofen "stands" on the surface (Figure 2(b)), while fenofibrate "lies flat" (Figure 2(d)). Despite these different molecular orientations, the obtained thicknesses consistently match the molecular heights, indicating that only the monolayer of molecules closest to the surface can dissolve in water. This provides strong evidence for the surface adsorption mechanism.

Next, the dissolution kinetics of the water-adsorbed drug molecules was studied. A very rapid dissolution rate was observed: over 90% of the fenofibrate API dissolved within the first 60 seconds (Figure 2(e)). This rate contrasts sharply with the hours-long dissolution times typically associated with mesoporous silicon dioxide formulations, as reported by Vallet-Regí (2004), indicating a very different dissolution mechanism.

In terms of polymer adsorption, Granick has already established that the adsorption amount $D^\sigma(t)$ follows an exponential decay model:

$$\frac{D^\sigma(t)}{D_0^\sigma} \sim \exp\left[-\left(\frac{t}{\tau_{off}}\right)^\beta\right]$$

The exponent $\beta$ is primarily influenced by the energy difference between solvent-surface and adsorbate-surface interactions. When $\beta = 1$, it indicates that the surface has a greater affinity for the solvent than for the adsorbate, leading to the surface releasing the adsorbate and attracting the solvent. In the experiments of the present invention, the drug API exhibited characteristics similar to short polymers. The undissolved API on the surface is consistent with the decay model, where the dissolution kinetics power is exactly 1, as shown in the inset of Figure 2(e).

Although measurements of dissolution quantity and rate have demonstrated adsorption and desorption behavior, a precise understanding of the underlying mechanisms remains elusive. To elucidate the fundamental mechanisms, first-principles density functional theory simulations were applied to capture the molecular interaction images. The simulations of the present invention revealed that, in a dry environment, the ultra-high density of silanol groups on the silica template surface can adsorb drug molecules and facilitate their desorption upon contact with water.

The adsorption process is first depicted in a dry environment, using ibuprofen as the example API. The volumetric density of ibuprofen allows estimating a surface density of 5 molecules per $nm^2$. This is consistent with the density of silanol groups on typical mesoporous silica surfaces, ranging from 4 to 6 groups per $nm^2$, averaging about 5 groups per $nm^2$. Therefore, in standard mesoporous silica, each ibuprofen molecule might form a hydrogen bond with one silanol group. According to DFT calculations, this one-to-one adsorption produces an enthalpy change of -58.17 kJ/mol, which is insufficient to overcome the evaporation enthalpy required to extract an ibuprofen molecule from the bulk, which is 80.3 kJ/mol. Thus, typical mesoporous silica cannot effectively adsorb ibuprofen to form a stable composite.

In contrast, the silica template of the present invention features a significantly higher density of silanol groups-up to four times higher than normal. This high density of silanol groups enhances the magnitude of the adsorption enthalpy. When an ibuprofen molecule interacts with two silanol groups, the adsorption enthalpy increases to -71 kJ/mol, approaching the evaporation enthalpy of ibuprofen. As the interactions strengthen, including each ibuprofen molecule with three or four silanol groups, the adsorption enthalpies (-84.82 kJ/mol and -81.00 kJ/mol) become strong enough to surpass the evaporation enthalpy of ibuprofen. This high density of silanol groups creates a "sticky" surface that effectively adsorbs drug molecules from the bulk, forming a monolayer, as previously shown in the data in Figure 2(a).

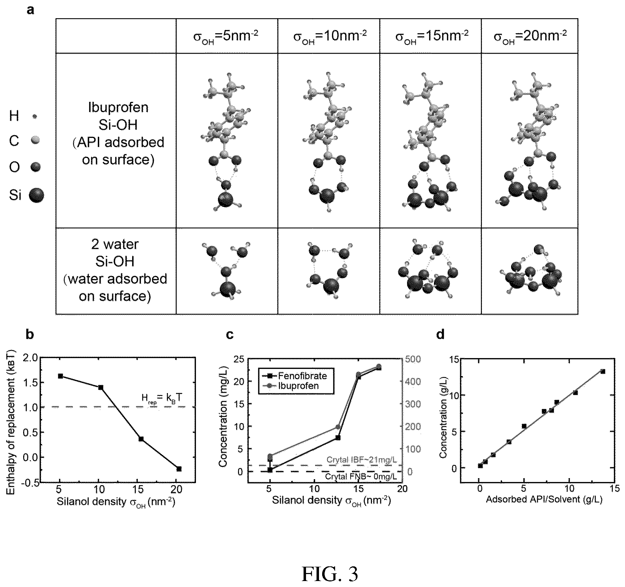

Figure 3 demonstrates an experimental evaluation of the effect of silanol density, examining the solubilization effects of materials with different silanol densities, and measuring the energy changes from API-silanol to water-silanol interactions through simulations.

a. The DFT optimized (B3LYP-D3/6-311++G**, SMD) geometric structures of ibuprofen-silanol and water-silanol interactions across four silanol densities ($\sigma\_OH$ = 5, 10, 15, and 20/nm^2). Silica clusters form hydrogen bonds (blue dashed lines) with different numbers of silanols, the first row with one ibuprofen molecule, and the second row with two water molecules. Considering the molecular volumes of ibuprofen, water, and amorphous silica, the silanol densities can be averaged in a single silica cluster as follows: $\sigma\_OH$ = 5/nm^2 for an isolated silanol, $\sigma\_OH$ = 10/nm^2 for a pair of twin silanols, $\sigma\_OH$ = 15/nm^2 for a pair of twin silanols plus an adjacent silanol, $\sigma\_OH$ = 20/nm^2 for two pairs of twin silanols. Atoms are arranged in descending order of size, with silicon (dark blue), oxygen (red), carbon (yellow), and hydrogen (green).

b. The solubilization effects of two APIs, ibuprofen (red) and fenofibrate (black), were evaluated using mesoporous silica materials with different $\sigma\_OH$ values.

c. The enthalpy change from IBF-silanol to water-silanol as a function of silanol density ($\sigma\_OH$), for the four silanol densities mentioned in (a).

d. Concentrations measured by UV-VIS spectroscopy (dissolved API/solvent) plotted against the total ratio of API to solvent. The API used here is ibuprofen, formulated with PE-07 at a 40% API to template ratio.

Figure 3(a) shows the most favorable adsorption configurations at different silanol densities, ranging from one-to-one to one-to-four (ibuprofen to silanol) interactions. Therefore, ultra-high silanol density is crucial for the stable adsorption of drug molecules onto the surface to form robust composite materials.

Adsorbing drug molecules onto surfaces is a key initial step, but facilitating their subsequent desorption and dispersion into water is equally important. The DFT simulations of the present invention again prove that the same ultra-high silanol density that makes adsorption possible under dry conditions is also crucial in aqueous environments. When the drug composite material is immersed in water, there is a competitive scenario for the adsorption of drug molecules and water molecules on the silica surface, depending on the difference in adsorption enthalpy values. Given that the area density of water is twice that of ibuprofen, it can be inferred that during the desorption process, two water molecules should replace one ibuprofen molecule.

On a typical silica surface with a silanol density of $\sigma\_OH = 5/nm^2$, the simulations of the present invention show that the adsorption enthalpy of two water molecules (Hw) is -14.73 kJ/mol, which is weaker than that of ibuprofen in a water environment (HIbu), which is -18.76 kJ/mol. Therefore, the enthalpy change associated with replacement, Hrep = Hw - HIbu = 4.03 kJ/mol (1.63kBT), is positive, and exceeds the thermal energy at room temperature, indicating that desorption and replacement on standard mesoporous silica are unlikely events. This may explain why incomplete drug release is commonly observed in typical silica, regardless of its pore structure, whether ordered mesoporous, microporous, or aerogel.

However, as the silanol density increases, both Hw and HIbu increase, with Hw increasing more significantly. Detailed data in Figure 3(b) show that as the silanol density $\sigma\_OH$ increases, the replacement enthalpy Hrep decreases, falling below room temperature dissipation $k_BT$ at about $\sigma\_OH = 12/nm^2$. This threshold indicates that at or above this silanol density, the desorption and dissolution processes of ibuprofen molecules are significantly facilitated. When the density reaches about $20/nm^2$, Hrep becomes negative, indicating that desorption and rapid dissolution are thermodynamically more favored. The lower row of Figure 3(a) displays the different molecular configurations of water molecules adsorbed at different silanol densities.

To validate the predictions derived from DFT simulations, a series of experimental studies were conducted on various surfaces with different silanol densities (methods). Two commercial mesoporous silica materials and three templates developed by the present invention were used. The two commercial products are mesoporous silica pharmaceutical excipients (Silsol® and Syloid®) with a silanol density of about $5/nm^2$; while the three templates developed by the present invention demonstrated higher silanol densities: $12.69/nm^2$, $15.01/nm^2$, and $17.34/nm^2$. To ensure the universality of the results of the present invention across different drugs, two different active pharmaceutical ingredients (APIs), ibuprofen and fenofibrate, were chosen. The same amount of each drug was loaded into the five types of silica materials using the same process. These drug composite materials were then dissolved in pure water, and the final dissolved fraction was measured.

Experimental results, as shown in Figure 3(c), indicate that the dissolution capacity is low when the silanol density of typical silica products is $5/nm^2$, while the templates of the present invention showed gradually improved solubility as $\sigma\_OH$ increases. Notably, a significant increase in solubility was observed at a silanol density of about $12/nm^2$. This observation is consistent with the theoretical point in Figure 3(b) where the replacement enthalpy (Hrep) equals room temperature dissipation ($k_BT$).

The excellent negative correlation between the solubility data in Figure 3(c) and the replacement enthalpy in Figure 3(b) emphasizes the effectiveness of DFT simulations. The results clearly demonstrate that higher silanol densities lead to lower replacement enthalpies, which facilitate the desorption of drug molecules and enhance drug solubility. Thus, the solubility measurements provide robust experimental support for the DFT calculations.

Furthermore, the present invention reveals an additional unique advantage: since the dissolution of drug molecules from the special surfaces of the present invention is driven by thermodynamic substitution enthalpy, more drug composite material can be continuously added to water to increase the drug concentration. This is demonstrated in Figure 3(d): the concentration of ibuprofen can be continuously increased to three orders of magnitude higher than its crystalline form, which is an unprecedented significant result! Although this ultra-high concentration represents a metastable state, this state remains stable for over 24 hours, which exceeds the usual time frame for the drug to take effect. Therefore, the technology of the present invention provides a unique advantage by achieving ultra-high drug concentrations, a property that could find practical potential in drug applications.

In summary, the development of silica templates with ultra-high silanol densities has significantly increased the solubility of drugs, easily achieving an enhancement of two to three orders of magnitude. This advancement is based on a new physical mechanism: the inherent adsorption and desorption processes of these engineered silica surfaces. This fundamental mechanism avoids the need for chemical modification of drug molecules and is applicable to a wide range of drug compounds. It is also efficient, cost-effective, scalable, and stable, all of which are highly valued in pharmaceutical manufacturing. Therefore, the method of the present invention has the potential to become a new generation of solubility enhancement platform technology and replace various state-of-the-art technologies.

Additionally, the PE - 07 template offers high-density interaction sites on its surface with its abundant stable silanol groups. These can be modified with various functional groups to accommodate a broader range of chemicals. This multifunctionality makes it potentially applicable in areas such as drug delivery, catalysis, controlled drug release, environmental remediation, and sensor development.

**Examples**

**Template Preparation and Drug Loading**

Templates were prepared using colloidal silica. The silica particles were nanosized, specifically 7 nanometers, 12 nanometers, and 22 nanometers, corresponding to PE-07, PE-12, and PE-22 respectively. To form the templates, these suspensions underwent a vacuum evaporation process at specific temperatures and pressures.

During the drug loading process, the following active pharmaceutical ingredients (APIs) were used: ibuprofen (Sigma-Aldrich®), ketoprofen, flurbiprofen, docetaxel, diclofenac, repaglinide, oxaprozin, isotretinoin, rapamycin, indomethacin, naproxen, simvastatin, trimebutine, fenofibrate, ramipril, paclitaxel, progesterone, and felodipine (all sourced from J&K Scientific®).

APIs were manually ground together with the appropriate templates. The ground mixture was then placed into molds and pressed using a hydraulic press, applying a pressure of 80MPa to form tablets. Subsequently, the tablets were baked at optimized temperatures for specific durations. The baking process aids in the relaxation of drug molecules and promotes effective interaction between the templates and APIs.

**Concentration Measurement**

In vitro dissolution tests were conducted using a homemade device designed according to the specifications of USP Apparatus 2, featuring a rotating paddle inside a cylindrical container at a speed of 50 RPM. The dissolution process began with the introduction of a single tablet into 500 mL of dissolution medium. After drug release, solution samples were collected at scheduled intervals to assess dissolution kinetics. The solubility profiles of 18 active pharmaceutical ingredients (APIs) were evaluated at the 6-hour time point. The solution was experimentally verified to have reached a stable, fully dissolved state at this time point. Afterwards, the solution samples were filtered using an Acrodisc® $0.2\mu$m syringe filter and the concentration was determined using a PerkinElmer® UV/Vis spectrophotometer L850. To establish the absorbance ($\varepsilon$), the linear coefficient linking UV/VIS absorption to concentration, calibration was performed by evaluating the absorption of a series of solutions with known concentrations.

**Pharmacokinetic Study**

The pharmacokinetic study involved 24 male C57BL/6J mice weighing 22-24 grams. Each of the four study groups consisted of six mice. The study included four groups: a 20% dose PE-07 formulated ibuprofen, 98% ibuprofen crystals (Sigma-Aldrich®), a 45.7% dose of ground Nurofen®, and pure PE-07 template as a control. The oral dosing concentrations were 50 mg/kg for PE-07 formulated ibuprofen and pure PE-07 template, 10 mg/kg for pure ibuprofen, and 21.9 mg/kg for Nurofen®. Apart from the control group, the mice were given the same amount of active ibuprofen (10mg/kg). Blood samples were collected at the following time points after a single dose: 15 minutes, 30 minutes, 1 hour, 2 hours, 4 hours, 8 hours, and 24 hours. The plasma concentrations were then measured using LC/MS analysis.

**Silanol Density Measurement**

At room temperature, the silanol groups on the surface of the template form hydrogen bonds and are in equilibrium with each other. However, at high temperatures, these groups undergo condensation and dehydration reactions. This characteristic allows for the quantification of silanols using Thermogravimetric Analysis (TGA). The amount of silanol per unit mass of the template was quantified using a TA Instruments® TGA550 system. The weight loss of the template was measured under gradually increasing temperature conditions. To eliminate the weight loss due to moisture in the pores, the weight measurement started at 200°C. By measuring the weight loss in the temperature range of 200-1000°C, the majority of silanol groups present on the template surface can be quantified (results in the Supplementary Information).

Ibuprofen and fenofibrate were selected as model active pharmaceutical ingredients (APIs), and templates were adjusted to investigate which properties of the templates play a dominant role in enhancing solubility. To explore this, colloidal suspensions with particle sizes of 5 nanometers, 7 nanometers, 12 nanometers, and 22 nanometers were used to prepare materials with four different silanol surface densities, specifically PE-05, PE-07, PE-12, and PE-22. Comparative experiments were conducted with commercially available silica mesoporous solubilizing excipients Syloid® and Silsol® (Grace®). The properties of the materials and their effects on enhancing the solubility of ibuprofen and fenofibrate are shown in Table 1.

**Table 1**

| | | PE-05 | PE-07 | PE-12 | PE-22 | Syloid®XDP | Silsol® | Crystalline drug |
|---|---|---|---|---|---|---|---|---|
| Specific Surface Area (m$^2$/g) | | 61.4 | 86.3 | 151.7 | 108.4 | 328.1 | 548.6 | - |
| Porosity | | 25.6% | 29.1% | 41.3% | 29.3% | 76.6% | 66.0% | - |
| Pore Diameter/nm | | 9.83 | 8.67 | 8.46 | 6.96 | 18.18 | 6.45 | - |
| Silanol Density(-OH/nm$^2$) | | 27.97 | 17.34 | 15.01 | 12.69 | 4~6 | 4~6 | - |
| Drug Dissolution Ratio | Fenofibrate | 59% | 55% | 50% | 18% | 1% | 6% | 0% |
| | Ibuprofen | 100% | 90% | 84% | 38% | 13% | 12% | 15% |

In Table 1, the specific surface area, average pore size, and porosity were characterized through the Brunauer-Emmett-Teller (BET) analysis of nitrogen gas adsorption isotherms. The silanol density, on the other hand, was measured through Thermogravimetric Analysis (TGA), which calculates the mass change of silicon materials as temperature varies. The mass loss below 200°C is due to the removal of physically adsorbed water, while the mass loss above 200°C results from the removal of surface hydroxyl groups. The Si-OH density is calculated by dividing the mass loss between 200°C and 1000°C by the specific surface area, as shown in the table. Notably, all the nanosized colloidal template fillers exhibit significantly higher surface Si-OH densities compared to Grace®'s mesoporous silica. In addition, the results show that smaller template sizes correspond to higher Si - OH densities, in the order of 5 nm > 7 nm > 12 nm > 22 nm.

After the five groups of silica templates were characterized, their capabilities to enhance the solubility of ibuprofen and fenofibrate were compared, as shown in the table. The Syloid silica show no significant differences compared to pure crystalline drugs in both APIs. Additionally, Silsol did not improve the dissolution of ibuprofen but slightly enhanced that of fenofibrate, consistent with the findings of Humbeeck. However, the PE series materials demonstrated significantly better performance in both APIs, despite having lower specific surface areas and porosities compared to Grace's silica. Moreover, materials with higher silanol densities showed better performance. This finding suggests that surface properties play a crucial role in enhancing dissolution, rather than pore size, porosity, or specific surface area. It can be reasonably inferred that the significant improvement in API dissolution is due to its interaction with the surface.

The surface silanol density is calculated by dividing the amount of silanol per unit mass by the template's specific surface area. The specific surface areas were measured using a Micromeritics® ASAP2020 (results in Supplementary Information). Before measurement, the templates were stored overnight in a vacuum oven at 120°C to remove moisture. This measurement is based on the Brunauer-Emmett-Teller (BET) method of nitrogen adsorption.

The silanol densities of PE-07, PE-12, and PE-22, as well as two typical types of silica, Silsol® and Syloid®, each with a silanol density of 5 OH/nm$^2$, were determined. Notably, Silsol® is specifically designed to enhance solubility.

**First-Principles Simulations**

First-principles simulations were conducted using the Gaussian16 software package. The calculations employed the B3LYP-D3 hybrid functional and the 6-311++G(d,p) basis set, which provides a good balance between computational cost and accuracy. The D3 dispersion correction was applied, as previous studies have shown that dispersion forces play a dominant role in the interaction between ibuprofen and silica surfaces.

The geometric structure of the silica clusters interacting with ibuprofen was optimized until the total enthalpy reached a minimum. The enthalpy H was calculated by adding the zero-point energy correction ($\Delta ZPE$) and thermal correction ($\Delta E_T$) to the BSSE-corrected electronic energy ($E^c$):

$$H = E^c + \Delta ZPE + \Delta E_T$$

The adsorption enthalpy $\Delta H_{Ibu}$ was determined by subtracting the sum of the enthalpies of ibuprofen ($H_{Ibu}$) and the silanol cluster ($H_{Si}$) from the enthalpy of the ibuprofen-silica combination ($H_{Ibu-Si}$):

$$\Delta H_{Ibu} = H_{Ibu-Si} - H_{Ibu} - H_{Si}$$

Similarly, the adsorption enthalpy for water dimer on silica $\Delta H_w$:

$$\Delta H_w = H_{w-Si} - H_w - H_{Si}$$

The adsorption process simulates the interaction of ibuprofen with silica clusters in a vacuum environment. For the dissolution process, a density-based solvation model (SMD) in water was used for simulation. This comparison involves analyzing the adsorption enthalpies of water dimers on silica and ibuprofen on silica.

This explanation, combined with specific/preferred embodiments, provides further detailed information about the invention but does not limit the invention to these descriptions alone. Those skilled in the art can make various alternatives or modifications to these described embodiments without departing from the scope of the invention. The reference terms "an embodiment," "some embodiments," "a preferred embodiment," "an example," "a specific example," or "some examples" in this description mean that the specific features, structures, materials, or characteristics described in connection with the embodiment or example are included in at least one embodiment or example of the invention. In this document, the schematic representation of these terms does not necessarily refer to the same embodiment or example. Moreover, the described specific features, structures, materials, or characteristics can be combined in any appropriate manner in one or more embodiments or examples. Without contradiction, those skilled in the art can combine and mix different described embodiments or examples and their features. Although the embodiments of the invention and its advantages have been described in detail, it should be understood that various changes, substitutions, and alterations can be made herein without departing from the scope of the invention as defined by the claims.

## Claims

1. A method for enhancing drug solubility, **characterized by** comprising the following steps:

   S1:raw material preparation: preparing a colloidal suspension of silicon dioxide in a solution, including silicon dioxide nanoparticles with diameters ranging from 2 to 100 nm;
   S2:preparation of high-density silanol group surface material: rapidly evaporating the solvent from the silicon dioxide colloidal suspension by controlling temperature and pressure, retaining an ultra-high density of silanol groups on the surface, enhancing the surface's affinity for drug molecules and water, and forming a porous structure to increase the specific surface area;
   S3: drug loading: mixing the active drug components with the high-density silanol group surface material, and load the drug molecules onto the material using a drug loading process.

2. The method for enhancing drug solubility according to claim 1, **characterized in that** the density of silanol groups on the surface of the silicon dioxide nanoparticles is greater than 10 OH/nm$^2$, to increase the adsorption capacity of the silicon dioxide nanoparticles for drug molecules.

3. The method for enhancing drug solubility according to claim 1 or 2, **characterized in that** in Step S2, the evaporation uses a vacuum evaporation process.

4. The method for enhancing drug solubility according to any one of claims 1 to 3, **characterized in that** in Step S3, the drug loading process includes grinding, pressing, and baking the mixture of the active drug component and the high-density silanol group surface material.

5. The method for enhancing drug solubility according to claim 4, **characterized in that** in Step S3, the pressing and baking process includes placing the ground mixture into a mold, pressing it with a hydraulic press to form a tablet, and subsequently baking the tablet at an optimized temperature for a set duration.

6. The method for enhancing drug solubility according to any one of claims 1 to 5, **characterized in that** the active drug components includes one or more of the following: fenofibrate, rapamycin, diclofenac, isotretinoin, trimetazidine, simvastatin, olaparib, rosiglitazone, indomethacin, felodipine, flurbiprofen, paclitaxel, docetaxel, naproxen, ibuprofen, progesterone, ketoprofen, and ramipril.

7. A drug composite material with enhanced solubility, **characterized in that** it is prepared by the method of any one of claims 1 to 6.

FIG. 1A

FIG. 1B

FIG. 2

FIG. 3

TRANSLATION

## INTERNATIONAL SEARCH REPORT

| International application No. |
|---|
| **PCT/CN2024/128187** |

| A. | CLASSIFICATION OF SUBJECT MATTER |
|---|---|

A61K 47/69(2017.01)i;  A61K9/14(2006.01)i;  A61K9/16(2006.01)i;  A61K47/02(2006.01)i

According to International Patent Classification (IPC) or to both national classification and IPC

| B. | FIELDS SEARCHED |
|---|---|

Minimum documentation searched (classification system followed by classification symbols)

IPC: A61K

Documentation searched other than minimum documentation to the extent that such documents are included in the fields searched

Electronic data base consulted during the international search (name of data base and, where practicable, search terms used)

CNTXT, WPABS, WPABSC, ENTXT, ENTXTC, DWPI, VCN, VEN, CJFD, CNKI, ISI-WEB OF SCIENCE, PUBMED, BING, STNext, 百度, BAIDU, 百度学术, BAIDU SCHOLAR, 读秀, DUXIU: 药容科技, 许卓, 二氧化硅, 胶体二氧化硅, 硅醇, 硅烷醇, 硅羟, 羟基, 多孔, 纳米, 蒸发, 快速蒸发, 真空, 研磨, 压, 压片, 烘烤, 加热, 难溶, 不溶, 溶解, 溶出, 布洛芬, 费诺贝特, SiO2, PHARMAEASE TECH, Silica, Colloidal silica, Silanol, Hydroxyl, Porous, Evaporae, Rapid evaporation, Grind, Press, Tablet, Bake, Heat, Poorly soluble, Dissolute, Ibuprofen, Fenofibrate

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | WO 2015065185 A1 (CAPRIGEMINI B.V.) 07 May 2015 (2015-05-07) claims 1-2, 7, 12, 16-17, and 19, and description, page 10, third-to-last paragraph, page 18, paragraphs 1-5, page 19, second-to-last line to fifth-to-last line, and embodiments 3 and 6 | 1-7 |
| A | CN 101829331 A (SHENYANG PHARMACEUTICAL UNIVERSITY) 15 September 2010 (2010-09-15) claims 1 and 9, and description, paragraphs [0017] and [0019] | 1-7 |
| A | CN 104968332 A (NEW JERSEY INSTITUTE OF TECHNOLOGY) 07 October 2015 (2015-10-07) claims 1, 4-8, 14, 18, 19, and 22, and description, paragraph [0074], and embodiments 2-4, 11, 17, and 22 | 1-7 |
| A | WO 2011154009 A1 (LIFECYCLE PHARMA A/S) 15 December 2011 (2011-12-15) claims 1-3 and 7 | 1-7 |

| ☑ Further documents are listed in the continuation of Box C. | ☑ See patent family annex. |
|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **09 July 2025** | **16 July 2025** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

TRANSLATION

## INTERNATIONAL SEARCH REPORT

| | International application No. |
|---|---|
| | **PCT/CN2024/128187** |

**C.    DOCUMENTS CONSIDERED TO BE RELEVANT**

| Category* | Citation of document, with indication, where appropriate, of the relevant passages | Relevant to claim No. |
|---|---|---|
| Y | MÜNZENBERG, J. et al. "Improving the Dissolution of Poorly Soluble APIs with Inorganic Solid Dispersions" <br> *Pharmaceutical Technology*, 01 April 2017 (2017-04-01), <br>       pages S12-S15 | 1-7 |
| A | KIM, J. M. et al. "Control of Hydroxyl Group Content in Silica Particle Synthesized by the Sol-Precipitation Process" <br> *Ceramics International*, Vol. 35, No. 3, 10 July 2008 (2008-07-10), <br>       pages 1015-1019 | 1-7 |
| A | SOLTYS, M. et al. "Drug Loading to Mesoporous Silica Carriers by Solvent Evaporation: A Comparative Study of Amorphization Capacity and Release Kinetics" <br> *International Journal of Pharmaceutics*, Vol. 607, 08 August 2021 (2021-08-08), <br>       pages 1-12 | 1-7 |

Form PCT/ISA/210 (second sheet) (July 2022)

## INTERNATIONAL SEARCH REPORT
### Information on patent family members

International application No.

**PCT/CN2024/128187**

| Patent document cited in search report | | | Publication date (day/month/year) | Patent family member(s) | | | Publication date (day/month/year) |
|---|---|---|---|---|---|---|---|
| WO | 2015065185 | A1 | 07 May 2015 | None | | | |
| CN | 101829331 | A | 15 September 2010 | None | | | |
| CN | 104968332 | A | 07 October 2015 | EP | 2916824 | A1 | 16 September 2015 |
| | | | | EP | 2916824 | A4 | 17 August 2016 |
| | | | | EP | 2916824 | B1 | 19 June 2019 |
| | | | | ES | 2745759 | T3 | 03 March 2020 |
| | | | | BR | 112015010704 | A2 | 11 July 2017 |
| | | | | BR | 112015010704 | B1 | 31 May 2022 |
| | | | | BR | 112015010704 | B8 | 14 June 2022 |
| | | | | JP | 2016503411 | A | 04 February 2016 |
| | | | | JP | 6460562 | B2 | 30 January 2019 |
| | | | | WO | 2014074808 | A1 | 15 May 2014 |
| | | | | US | 2015297521 | A1 | 22 October 2015 |
| | | | | US | 9801820 | B2 | 31 October 2017 |
| | | | | AR | 093449 | A1 | 10 June 2015 |
| | | | | DK | 2916824 | T3 | 30 September 2019 |
| WO | 2011154009 | A1 | 15 December 2011 | None | | | |

Form PCT/ISA/210 (patent family annex) (July 2022)